Europäisches Patentamt
European Patent Office
Office européen des brevets

(19) (11) Publication number: **0 301 465 A1**

(12) **EUROPEAN PATENT APPLICATION**

(21) Application number: **88111995.2**

(51) Int. Cl.[4]: **C07D 455/02 , //A61K31/435**

(22) Date of filing: **26.07.88**

(30) Priority: **28.07.87 JP 189570/87**

(43) Date of publication of application: **01.02.89 Bulletin 89/05**

(84) Designated Contracting States: **AT BE CH DE ES FR GB IT LI LU NL SE**

(71) Applicant: **FUJISAWA PHARMACEUTICAL CO., LTD.**
**3, Doshomachi 4-chome Higashi-ku**
**Osaka-shi Osaka 541(JP)**

(72) Inventor: **Kitamura, Satoshi**
**45, B-814, Yamadaminami**
**Suita-shi Osaka 565(JP)**
Inventor: **Okamoto, Yoshihiko**
**2-1-27, Takawashi**
**Habikino-shi Osaka 583(JP)**
Inventor: **Tada, Toshiji**
**3-4-28, Nishikouji**
**Minoo-shi Osaka 562(JP)**
Inventor: **Momonaga, Masashi**
**1-6-12, Umamikita Koryo-cho**
**Kitakatsuragi-gun Nara 635(JP)**

(74) Representative: **Türk, Gille, Hrabal**
**Bruckner Strasse 20**
**D-4000 Düsseldorf 13(DE)**

(54) **Crystalline monohydrate of sodium N-(1H-tetrazol-5-yl)-1-phenoxy-4H-quinolizin-4-one-3-carboxamide.**

(57) The present invention relates to a crystalline monohydrate of sodium N-(1H-tetrazol-5-yl)-1-phenoxy-4H-quinolizin-4-one-3-carboxamide which has pharmaceutical actions such as antiallergic and antiulcer actions and to processes for preparation thereof.

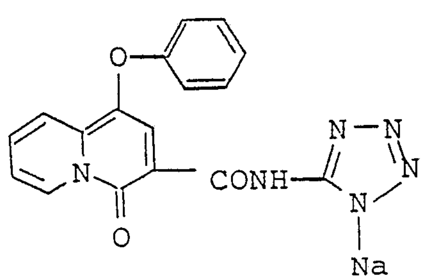

· $H_2O$

EP 0 301 465 A1

# CRYSTALLINE MONOHYDRATE OF SODIUM N-(1H-TETRAZOL-5-YL)-1-PHENOXY-4H-QUINOLIZIN-4-ONE-3-CARBOXAMIDE

The present invention relates to a crystalline monohydrate of sodium N-(1H-tetrazol-5-yl)-l-phenoxy-4H-quinolizin-4-one-3-carboxamide which has pharmaceutical actions such as antiallergic and antiulcer actions.

Sodium N-(1H-tetrazol-5-yl)-1-phenoxy-4H-quinolizin-4-one-3-carboxamide, which has the following structural formula, was first described by the present applicant in U.S. Patent No. 4,650,804 and, as such, is a known compound.

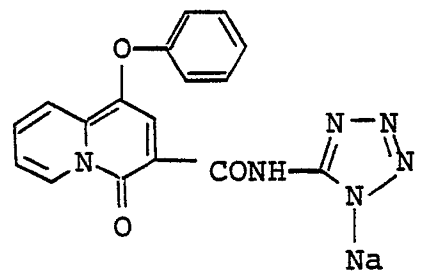

This compound has been found to have excellent antiallergic and antiulcer activities and its application to the human body as a drug for internal use has been contemplated. In this specification, the above chemical compound is referred to briefly as QC compound.

QC compound, as produced by the production process described in the above Patent, is anhydride as evident, from the description on column 42 of the same Patent and further stays in an amorphous form by the scanning electron micrograph described below. However, the amorphous form has the disadvantages that it is, in general,bulky and difficulty fusible on a constant temperature and so unstable, therefore, it does not appear to be suitable for a pharmaceutical product and is not easy to handle in the pharmaceutical preparations and storage. According to intensive study by the present inventors, they found that this compound can crystallize as its hydrate depending upon the processing conditions or environment and the crystal can assume either the form of monohydrate or the form of tetrahydrate. Further, they found that the monohydrate form is superior to the tetrahydrate form in stability and antihygroscopicity.

The stability and antihygroscopicity of the crystalline monohydrate of QC compound are described below, reference being made to the course of development of the present invention.

According to the production examples for QC compound in the above-mentioned U.S. Patent No. 4,650,804 , lyophilization is carried out at a final stage of its production. While this implies that the resulting compound is amorphous and has no crystalline water, this assumption is supported by the elemental analysis data shown.

Now, in the preparation of QC compound in large lots for purposes of physicochemical and pharmacological investigations, the compound crystallized from water-ethanol at the final purification stage was found to contain 4 molecules of crystalline water (theoretical water content : 16%). However, because of its weak bonding power, this crystalline water is readily released under inappropriate storage conditions and this release of water molecules was found to result in physicochemical changes of the order causing changes in IR spectrum and thin-layer chromatogram (hereinafter, TLC). The findings suggested the existence of some crystalline form more stable than the form containing 4 molecules of crystalline water (which will hereinafter be referred to sometimes as type A crystalline form). With this assumption as an impetus, type A crystalline form was stored under various conditions and the resulting changes were investigated. As a result, the existence of a crystalline form containing one molecule of crystalline water (theoretical water content : 4.6%) (hereinafter referred to sometimes as type B crystalline form) was discovered. It was also found that depending on storage conditions, the crystalline water may be completely released or, conversely, the crystals may absorb moisture to undergo change to a higher water content. Thus, according to the presence or absence, or the amount, of crystalline water, QC compound can be classified into amorphous form, type A crystalline form and type B crystalline form. Moreover, there seemed to be differences among them in physicochemical properties. Therefore, we conducted several experiments for elucidating the mutual reversibility, stability and other characteristics of the above three forms. Results of such experiments are set forth below, with some explanations.

Experiment 1 : Mobility of crystalline water under storage conditions

(A) Mobility of crystalline water at elevated temperature in dry atmosphere in an open system

When type A crystalline form (water content 16:1%) was stored in atmospheric air (relative humidity 0-5%) (hereinafter relative humidity is referred to sometimes as RH) at 80°C and 100°C in an open container, the water content was reduced to about 5% at the former temperature and about 1% at the latter temperature, both in one day, and no further change was observed even when the crystalline forms were kept stored for a total of 2 weeks under the same conditions. Then, when heating was discontinued and the crystals were placed back under room temperature conditions (exposed to atmospheric air), the original water content was quickly restored and the IR spectrum in this state was not different from that of the original samples.

These experimental results suggested that while type A crystalline form includes 4 water molecules in the gaps existed in crystal lattices constituted by QC compound molecules, 3 of the 4 water molecules are liable to be released at 80°C and the remaining one water molecule is liable to be released at 100°C. It was also presumed that the ingoing and outgoing of such crystalline water are not accompanied by any fundamental change in crystal structure.

(B) Mobility of crystalline water during storage in a closed system

Type A crystalline form (water content 16.1%) was stored in a closed container (initial relative humidity about 50%) at 50°C for 3 months and, then, the IR spectrum was determined. As a result, an IR absorption pattern obviously distinct from that of type A crystalline form was observed, suggesting a transformation of type A crystalline form to type B crystalline form. However, the apparent water content of 8.55% was higher than the theoretical water content of 4.6% for type B crystalline form, suggesting that some of the outgoing water remained, physically attached to the surface of crystals. Therefore, the same samples were further stored under various conditions. The results were that the crystals stored at 25°C with 93% RH for 5 days assumed a water content of 5.25% and their IR spectrum was not different from that of type B crystalline form. Although no detailed account can be made for departure of attached water under such high-humidity conditions, the observed release of water probably took place as the attached water was made easier to migrate partly because of the open conditions under which the crystals were placed and partly because of the affinity of the attached water for atmospheric moisture.

(C) Mobility of crystalline water under elevated temperature and elevated humidity conditions

When type A crystalline form (water content 16.1%) was stored at 37°C (11%, 48% and 89% RH), substantially no change occurred in water content, nor was found any appreciable change in IR spectrum, under either of the two humidity conditions of 48% and 89% RH. On the other hand, under the humidity conditions of 11% RH, the water content decreased to 9.6% in 2 weeks but no change was noted in the crystal lattice of type A crystalline form.

When samples were stored at 60°C (11%, 44% and 88% RH), the water content decreased to about 5% within 24 hours and remained at the level of about 5% even after 2 weeks. Thereafter, when the same samples were placed in the laboratory room atmosphere (about 25°C, about 50% RH), the water content of the sample which had been stored at 11% RH returned to 15.3%, with the IR spectrum of the sample resuming the pattern of original type A crystalline form but all the other samples retained the water level of about 5% and showed IR spectra distinct from the spectrum of type A crystalline form.

From these results of experiments (A) to (C), it was clear that the transformation from type A crystalline form to type B crystalline form not only requires the release of three molecules of water but calls for some heat and moisture. It was thus found that QC compound exists not only in the amorphous form but also in the tetrahydrate of type A crystalline form and the monohydrate of type B crystalline form.

According to the above experimenal findings and the examples described later, type A crystalline form can be prepared by using a mixture of water and ethanol, for instance, as the solvent for crystallization at the final stage of production of QC compound. When a mixture of water and ethanol is employed, the volume proportion of water is preferably not less than 20%, more preferably 20 to 60%, and most

preferably 40%.

Type B crystalline form can be prepared by placing type A crystalline form or amorphous form under elevated temperature and humidity conditions to induce each transformation of crystal or crystallization, and the condition preferred for this purpose are generally 40 to 80 °C and not less than 30% RH.

As another approach to prepare type B crystalline form, it can be prepared by using a mixture of water and isopropyl alcohol or a mixture of water and acetone, for instance, as the solvent for crystallization at the final stage of production of QC compound. In each solvent, the volume proportion of water is preferably not less than 20%, more preferably 20 to 60%. The most preferable solvent for crystallization is a mixture of water and isopropyl alcohol (40:60). The above approach also includes a method that type A crystalline form is once prepared by using a mixture of water and ethanol as the solvent for crystallization, and then type B crystalline form is prepared by using water-isopropyl alcohol for recrystallization. With regard to the method to prepare type B crystalline form, it is not restricted to the above methods and includes any method capable of obtaining said crystalline form.

Experiment 2 : Physicochemical properties

The physicochemical properties of the aforesaid amorphous, type A crystalline and type B crystalline forms will be collectively described below.

Fig. 1 shows the IR absorption spectra, Fig. 2 shows the X-ray powder diffraction patterns, Fig. 3 shows the moisture absorption equilibrium patterns, Fig. 4 shows the thermal analysis curves, and Fig. 5 shows the scanning electron micrographs. In each view, (A) denotes type A crystalline form, (B) type B crystalline form and (C) amorphous form.

It is clear from these data that type A and type B crystalline form show good crystallinity and that while the former crystals are columns (crystalline powder in appearance), the latter are platelets (well-defined crystals in appearance). In contrast, the amorphous form shows little evidence of crystallinity and appears to be a yellow powder. All these forms melt at 250 °C and higher temperatures (decomposition) and type A crystalline form begins to lose water at a comparatively early stage of temperature increase (40-150 °C), while type B crystalline form begins to release water at a relatively high temperature (about 180-220 °C) suggesting that type B crystalline form is superior to type A crystalline form in thermal stability. The results of the moisture absorption equilibrium experiment indicate that whereas the changes in water content in response to changes in relative humidity are remarkable in type A crystalline and amorphous forms, type B crystalline form changes little in water content, suggesting a superior antihygroscopicity of type B crystalline form.

Experiment 3 : Stability

The amorphous, type A crystalline and type B crystalline forms were subjected to a stability test. The results are set forth in Table 1.

Table 1

| Test item | | Samples and Storage Conditions | | | | | | | | | | | |
|---|---|---|---|---|---|---|---|---|---|---|---|---|---|
| | | Amorphous form | | | | Type A crystalline form | | | | Type B crystalline form | | | |
| | | Initial | 80° C x 5 days (closed container) | 37° C, 89% RH, 5 days | 30,000 Lux, 5 days | Initial | 80° C x 5 days (closed container) | 37° C, 89% RH, 5 days | 30,000 Lux, 5 days | Initial | 80° C x 5 days (closed container) | 37° C, 89% RH, 5 days | 30,000 Lux, 5 days |
| Appearance | | Yellow powder | Yellow powder | Yellow powder | Yellow powder (*1) | Yellow crystalline powder | Yellow crystalline powder | Yellow crystalline powder | Yellow crystalline powder (*1) | Yellow crystals | Yellow crystals | Yellow crystals | Yellow crystals |
| Water content (%) (*5) | | 7.00 | 6.86 | 6.53 | 5.11 | 14.9 | 13.7 | 16.1 | 12.9 | 5.16 | 4.87 | 5.09 | 4.87 |
| Assay (%) (*2) | | 100 | 101.4 | 100.8 | 42.8 | 100 | 100.3 | 101.2 | 36.1 | 100 | 99.2 | 99.9 | 97.7 |
| IR spectrum | | - | Slight change | Change (*3) | Change (*4) | - | Change (*3) | No change | Slight change | - | No change | No change | No change |
| TLC | Method A | - | No change | No change | Change | - | No change | No change | Change | - | No change | No change | Slight change |
| | Method B | - | No change | No change | Change | - | No change | No change | Change | - | No change | No change | Slight change |

(*1) The surface had a faintly brown tinge.
(*2) The assay value represents the % residue to the initial value.
(*3) The IR spectrum was in agreement with that of type B crystalline form.
(*4) A change presumably due to decomposition was noted.
(*5) The water in the amorphous form represents the water physically attached to the surface.

Referring, first, to appearance, both the amorphous and type A crystalline forms assumed a slight brown tinge under exposure to light at 30,000 Lux but type B crystalline form showed no change at all under the same conditions.

In regard to change in water content, too, type B crystalline form was most stable. In contrast, the amorphous form showed a remarkable change in water content under exposure to light at 30,000 Lux and the type A crystalline form also did so under the conditions of 37°C, 89% RH and under exposure to light at 30,000 Lux.

The assay data indicate clearly that the amorphous form and the type A crystalline form are not satisfactory in terms of stability under exposure to light and are considerably less stable than the type B crystalline form.

Analysis by IR absorption spectrometry also revealed a change in the amorphous form which was suggestive of degradation under exposure to light, indicating that both amorphous and type A crystalline forms are labile.

The TLC performed for detection of degradation products revealed changes in all the forms of QC compound stored under exposure to light but the change of type B crystalline form seemed not appreciable.

Experiment 4 : Stability after formation with excipients

Since QC compound is under development as a drug for internal use, its stability in formation with various common excipients for oral preparations was investigated. The results are summarized in Table 2.

Table 2

| Test Item | | Amorphous form | | | Type A crystalline form | | | Type B crystalline form | | |
|---|---|---|---|---|---|---|---|---|---|---|
| | | Lactose | Crystalline cellulose | Corn starch | Lactose | Crystalline cellulose | Corn starch | Lactose | Crystalline cellulose | Corn starch |
| Appearance | | Yellow powder | Yellow powder | Yellow powder | Yellow-brown powder | Yellow powder | Yellow powder | Yellow powder | Yellow powder | Yellow powder |
| Assay | | 97.5 | 101.9 | 99.9 | 69.8 | 98.4 | 101.3 | 101.0 | 100.2 | 99.5 |
| TLC | Method A | + | + | - | + | + | - | + | + | - |
| | Method B | + | + | - | + | + | - | + | + | - |
| | Method C | - | - | - | - | - | - | - | - | - |

(Note 1) The assay value represents the % residue to the initial value

(Note 2) In the TLC column, - stands for no change and + stands for a slight change.

In the formations with lactose and with crystalline cellulose, irrespective of forms of QC compound, degradation products were invariable detected by TLC but it was in the case of formation of type A crystalline form with lactose that a serious problem was suggested by assay data. Furthermore, the formation of the amorphous form with lactose and the formation of type A crystalline form with crystalline cellulose also generated assay data which cannot be disregarded in terms of stability. In contrast, type B crystalline form showed no problem in terms of assay data.

The following preparation and examples are given for purpose of illustrating the present invention.

<u>Preparation 1</u>

In a mixture of ethanol (33.9 ℓ) and water (22.6 ℓ) was dissolved of sodium N-(1H-tetrazol-5-yl)-1-phenoxy-4H-quinolizin-4-one-3-carboxamide (8.70 kg) under warming and under reflux at 80° C. Then, carbon powder (174 g) was added thereto and the mixture was refluxed with stirring at 79-80° C for 0.5 hour. The mixture was then filtered when hot and the carbon powder was washed with 60% aqueous ethanol (13 ℓ). The filtrate and washings were combined, and after dissolving the precipitate by refluxing, the solution was cooled to 10° C (cooling rate : 1° C/min) and stirred for ripening for 1 hour at the same temperature. The precipitate was recovered by centrifugation, washed with ethanol (17.4 ℓ) and dried. The procedure gave crystalline tetrahydrate of sodium N-(1H-tetrazol-5-yl)-1-phenoxy-4H-quinolizin-4-one-3-carboxamide (type A crystalline form) (8.62 kg) with a water content of 14.9%.

<u>Example 1</u>

In a mixture of isopropyl alcohol (34 ℓ) and water (22.7 ℓ) was dissolved crystalline tetrahydrate of sodium N-(1H-tetrazol-5-yl)-1-phenoxy-4H-quinolizin-4-one-3-carboxamide (type A crystalline form) (7.08 kg) under warming. Then, carbon powder was added thereto and the mixture was refluxed with stirring at 79-80° C for 0.5 hour. The mixture was then filtered when hot and the carbon powder was washed with 60% aqueous isopropyl alcohol. The filtrate and washings were combined, cooled and stirred for ripening for 1 hour. The precipitate was recovered by centrifugation, washed with isopropyl alcohol and dried. The procedure gave crystalline monohydrate of sodium N-(1H-tetrazol-5-yl)-1-phenoxy-4H quinolizin-4-one-3-carboxamide (type B crystalline form) (5.62 kg) with a water content of 4.9%.

(i) In its X ray powder diffraction pattern, it is characterized by peaks at about 5.6°, 11.3°, 18.1° and 28.0°

(ii) In its infrared absorption spectrum (Nujol), it is characterized by absorption peaks at about 3505, 3340, 3250, 1656, 1554, 1068 and 859 $cm^{-1}$

<u>Example 2</u>

In a mixture of isopropyl alcohol (63.5 ℓ) and water (42.3 ℓ) was dissolved of sodium N-(1H-tetrazol-5-yl)-1-phenoxy-4H-quinolizin-4-one-3-carboxamide (13.22 kg) under reflux at 80-81° C. Then, carbon powder (264 g) was added thereto and the mixture was refluxed with stirring at 80° C for 0.5 hour. The mixture was then filtered when hot and the carbon powder was washed with 60% aqueous isopropyl alcohol. The filtrate and washings were combined, and after dissolving the precipitate by refluxing, the solution was cooled to 10° C (cooling rate : 1° C/min.) and stirred for ripening for 1 hour at the same temperature. The precipitate was recovered by centrifugation, washed with isopropyl alcohol (26.4 ℓ) and dried. The procedure gave crystalline monohydrate of sodium N-(1H-tetrazol-5-yl)-1-phenoxy-4H-quinolizin-4-one-3-carboxamide (type B crystalline form) (11.39 kg) with a water content of 4.9%.

<u>Example 3</u>

Sodium N-(1H-tetrazol-5-yl)-1-phenoxy-4H-quinolizin-4-one-3-carboxamide (amorphous form) (205.4 mg) was put in a glass bottle without stopper, which was then kept in a desiccator (75% RH) containing saturated aqueous sodium chloride at 60° C for 18 hours, and dried. The procedures gave crystalline monohydrate of sodium N-(1H-tetrazol-5-yl)-1-phenoxy-4H-quinolizin-4-one-3-carboxamide (type B crystalline form) (200.8 mg) with a water content of 4.9 %.

Example 4

Crystalline tetrahydrate of sodium N-(1H-tetrazol-5-yl)-1-phenoxy-4H-quinolizin-4-one-3-carboxamide (type A crystalline form) (498.6 mg) was put in a glass bottle without stopper, which was then kept in a desiccator (75% RH) containing saturated aqueous sodium chloride at 60 °C for 18 hours, and dried. The procedures gave crystalline monohydrate of sodium N-(1H-tetrazol-5-yl)-1-phenoxy-4H-quinolizin-4-one-3-carboxamide (type B crystalline form) (446.5 mg) with a water content of 4.9%.

The type B crystalline form of the invention is superior to the amorphous form and the type A crystalline form in antihygroscopicity and stability and may therefore be provided as a pharmaceutical product which is easy to handle.

Brief Description of the Drawings

Fig. 1 shows IR absorption spectra, Fig. 2 shows X-ray powder diffraction patterns, Fig. 3 shows moisture absorption equilibrium graphs, Fig. 4 shows thermal analysis curves, and Fig. 5 shows scanning electron micrographs showing the appearance of crystals.

## Claims

1. Crystalline monohydrate of sodium N-(1H-tetrazol-5-yl)-1-phenoxy-4H-quinolizin-4-one-3-carboxamide.

2. The crystalline substance of claim 1 which has the following physical properties :

(i) in its X ray powder diffraction pattern, it is characterized by peaks at about 5.6°, 11.3°, 18.1° and 28.0°;

(ii) in its infrared absorption spectrum (Nujol), it is characterized by absorption peaks at about 3505, 3340, 3250, 1656, 1554, 1068, 859 $cm^{-1}$.

3. Crystalline monohydrate of sodium N-(1H-tetrazol-5-yl)-1-phenoxy-4H-quinolizin-4-one-3-carboxamide which is obtainable by crystallizing sodium N-(1H-tetrazol-5-yl)-1-phenoxy-4H-quinolizin-4-one-3-carboxamide from a mixture of water and isopropyl alcohol.

4. Crystalline monohydrate of sodium N-(1H-tetrazol-5-yl)-1-phenoxy-4H-quinolizin-4-one-3-carboxamide which is obtainable by recrystallizing crystalline tetrahydrate of sodium N-(1H-tetrazol-5-yl)-1-phenoxy-4H-quinolizine-4-one-3-carboxamide from a mixture of water and isopropyl alcohol.

5. The crystalline substance of claim 3 or 4, wherein the proportion of water in a mixture of water and isopropyl alcohol is 20 to 60% by volume.

6. The crystalline substance of claim 5, wherein the proportion of water in a mixture of water and isopropyl alcohol is about 40% by volume.

7. Crystalline monohydrate of sodium N-(1H-tetrazol-5-yl)-1-phenoxy-4H-quinolizin-4-one-3-carboxamide which is obtainable by crystallizing sodium N-(1H-tetrazol-5-yl)-1-phenoxy-4H-quinolizin-4-one-3-carboxamide under 40° to 80° C and not less than 30% in relative humidity.

8. Crystalline monohydrate of sodium N-(1H-tetrazol-5-yl)-1-phenoxy-4H-quinolizin-4-one-3-carboxamide which is obtainable by transforming crystalline tetrahydrate of sodium N-(1H-tetrazol-5-yl)-1-phenoxy-4H-quinolizin-4-one-3-carboxamide under 40° to 80° C and not less than 30% in relative humidity.

9. A process for preparing crystalline monohydrate of sodium N-(1H-tetrazol-5-yl)-1-phenoxy-4H-quinolizin-4-one-3-carboxamide which comprises crystallizing sodium N-(1H-tetrazol-5-yl)-1-phenoxy-4H-quinolizin-4-one-3-carboxamide from a mixture of water and isopropyl alcohol.

10. A process for preparing crystalline monohydrate of sodium N-(1H-tetrazol-5-yl)-1-phenoxy-4H-quinolizin-4-one-3-carboxamide which comprises recrystallizing crystalline tetrahydrate of sodium N-(1H-tetrazol-5-yl)-1-phenoxy-4H-quinolizin-4-one-3-carboxamide from a mixture of water and isopropyl alcohol.

11. The process of claim 9 or 10, wherein the proportion of water in a mixture of water and isopropyl alcohol is 20 to 60% by volume.

12. The process of claim 11, wherein the proportion of water in a mixture of water and isopropyl alcohol is about 40% by volume.

13. A process for preparing crystalline monohydrate of sodium N-(1H-tetrazol-5-yl)-1-phenoxy-4H-quinolizin-4-one-3-carboxamide which comprises crystallizing sodium N-(1H-tetrazol-5-yl)-1-phenoxy-4H-quinolizin-4-one-3-carboxamide under 40° to 80° C and not less than 30% in relative humidity.

14. A process for preparing crystalline monohydrate of sodium N-(11H-tetrazol-5-yl)-1-phenoxy-4H-quinolizin-4-one-3-carboxamide which comprises transforming crystalline tetrahydrate of sodium N-(1H-tetrazol-5-yl)-1-phenoxy-4H-quinolizin-4-one-3-carboxamide under 40° to 80° C and not less than 30% in relative humidity.

15. A process for preparing crystalline monohydrate of sodium N-(1H-tetrazol-5-yl)-1-phenoxy-4H-quinolizin-4-one-3-carboxamide which comprises

(a) crystallizing sodium N-(1H-tetrazol-5-yl)-1-phenoxy-4H-quinolizin-4-one-3-carboxamide from a mixture water and ethanol, and then

(b) recrystallizing the resultant crystalline tetrahydrate of sodium N-(1H-tetrazol-5-yl)-1-phenoxy-4H-quinolizin-4-one-3-carboxamide from a mixture water and isopropyl alcohol.

Fig. 1

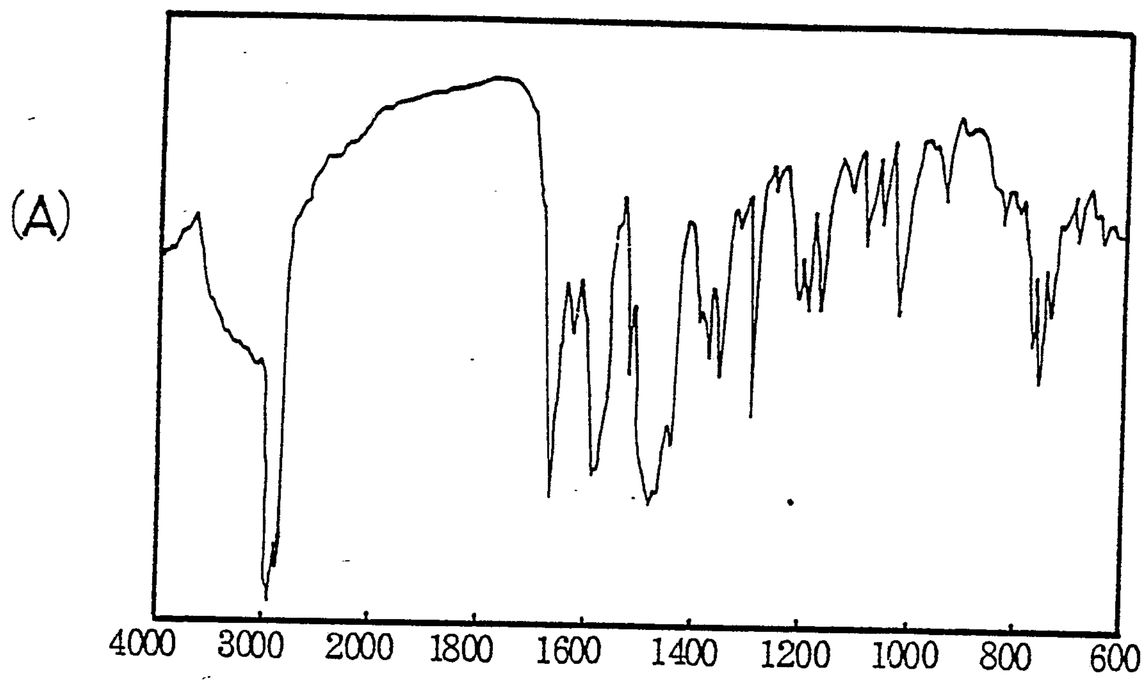

Fig. 1

(B)

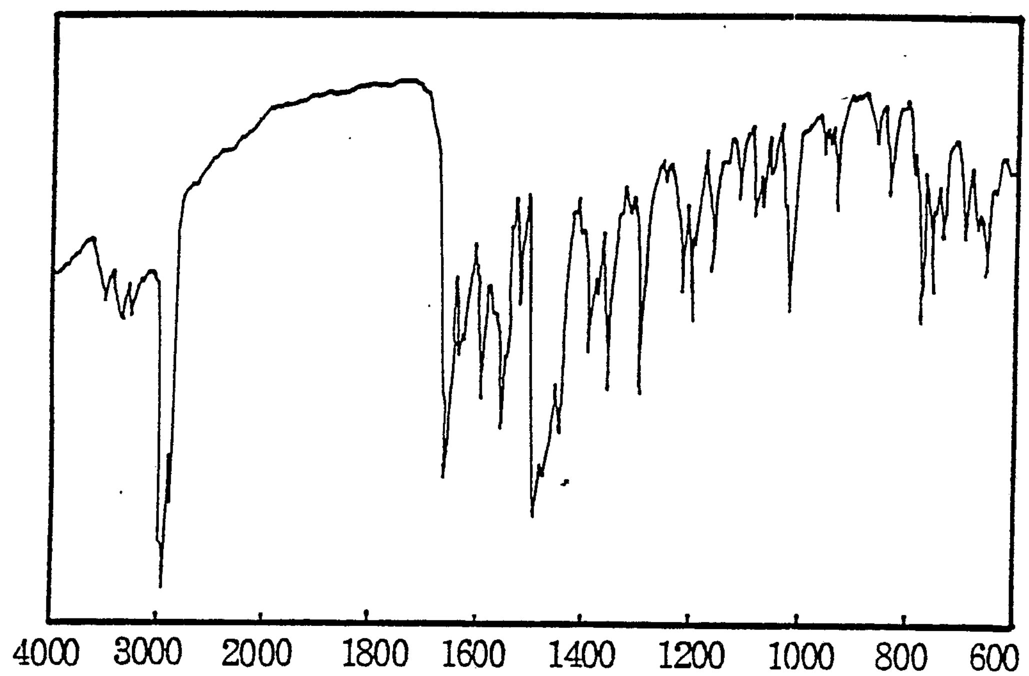

Fig. 1

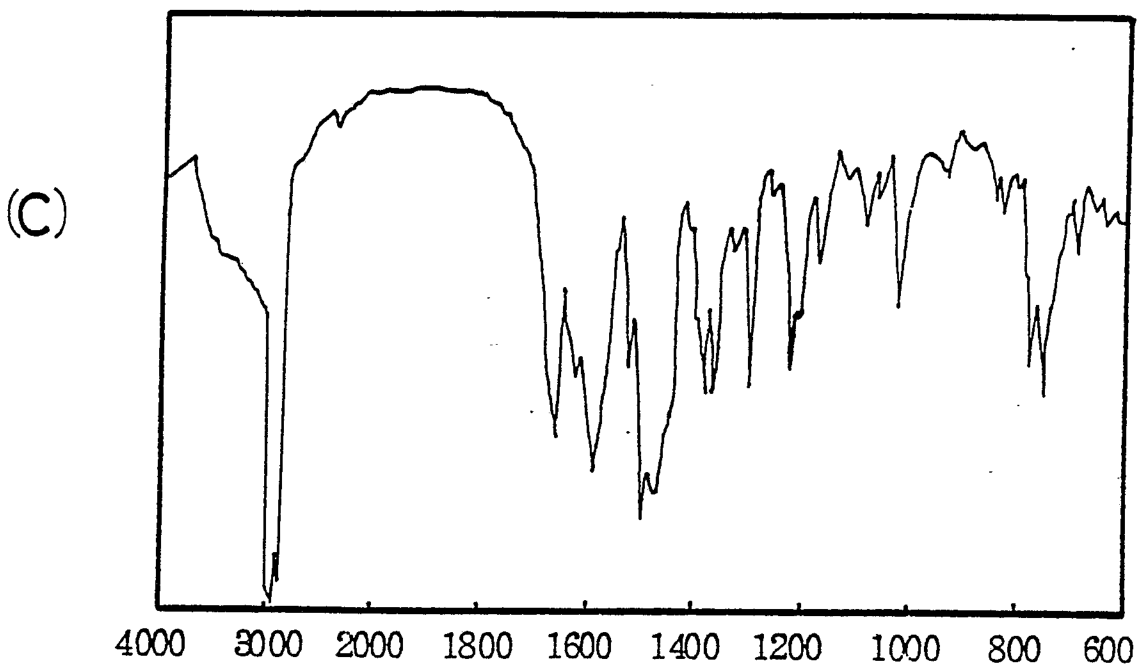

Fig. 2

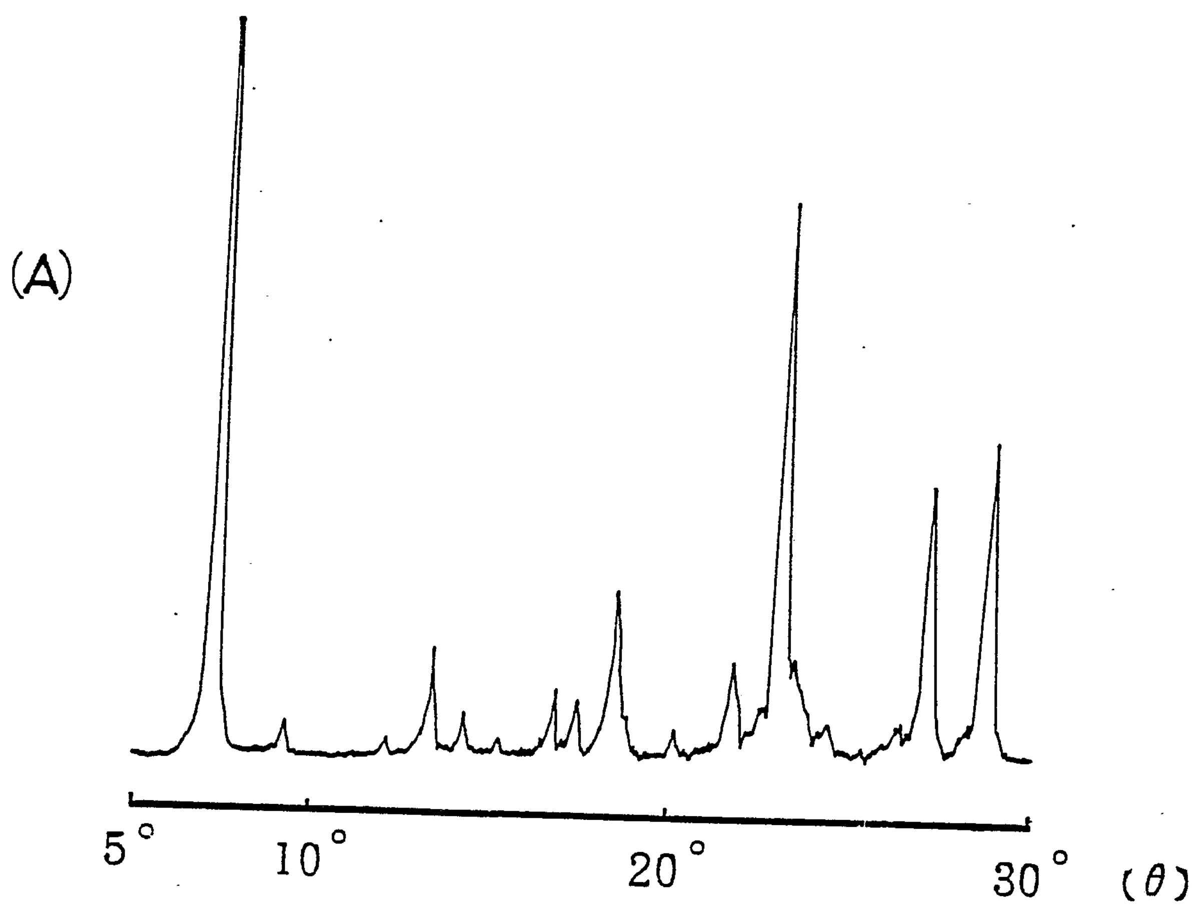

Fig. 2

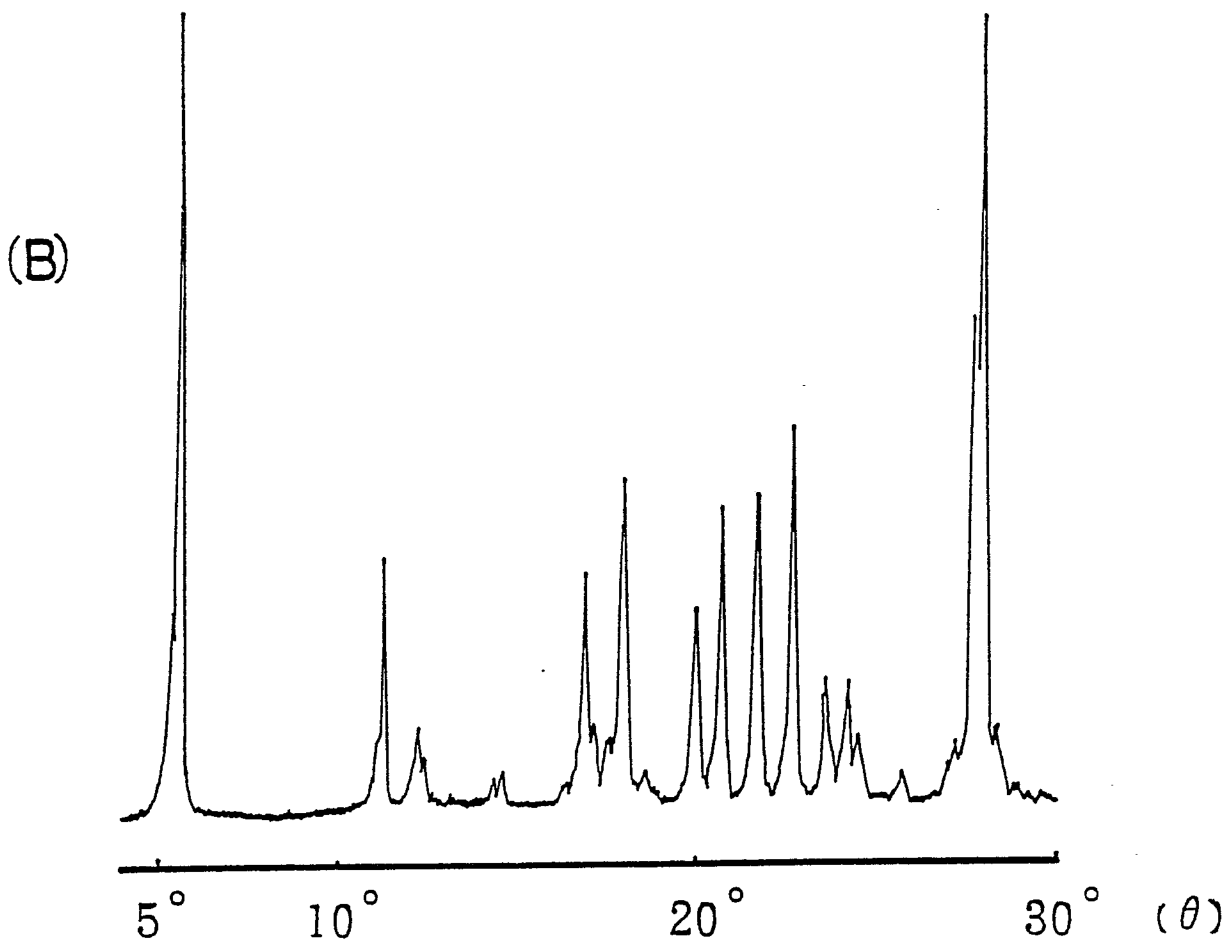

Fig. 2

(C)

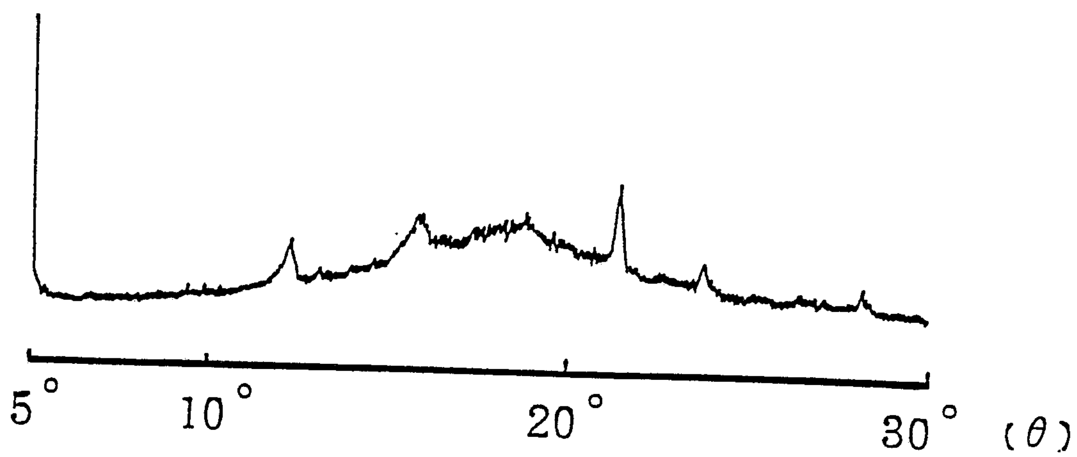

Fig. 3

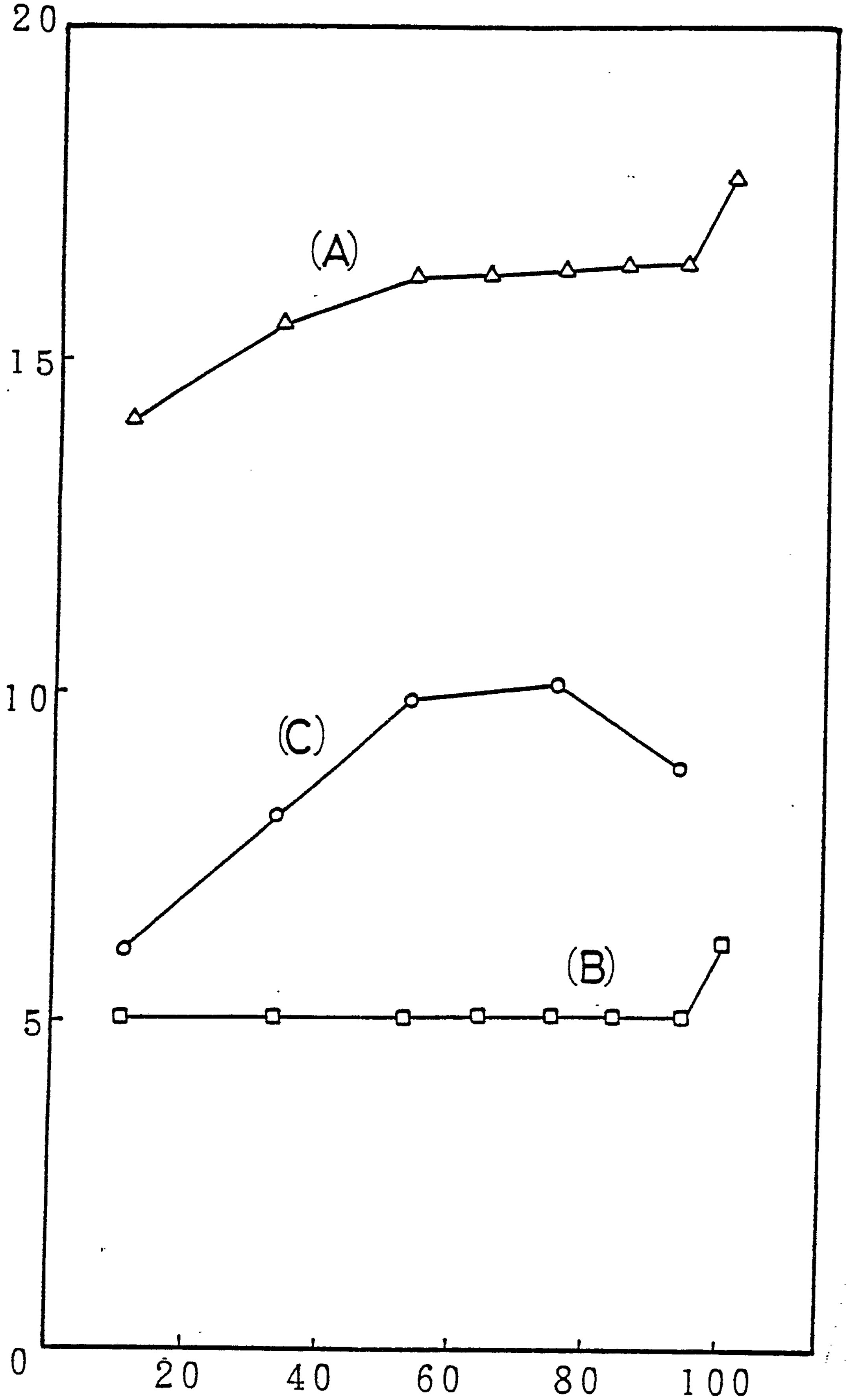

Fig. 4

(A)

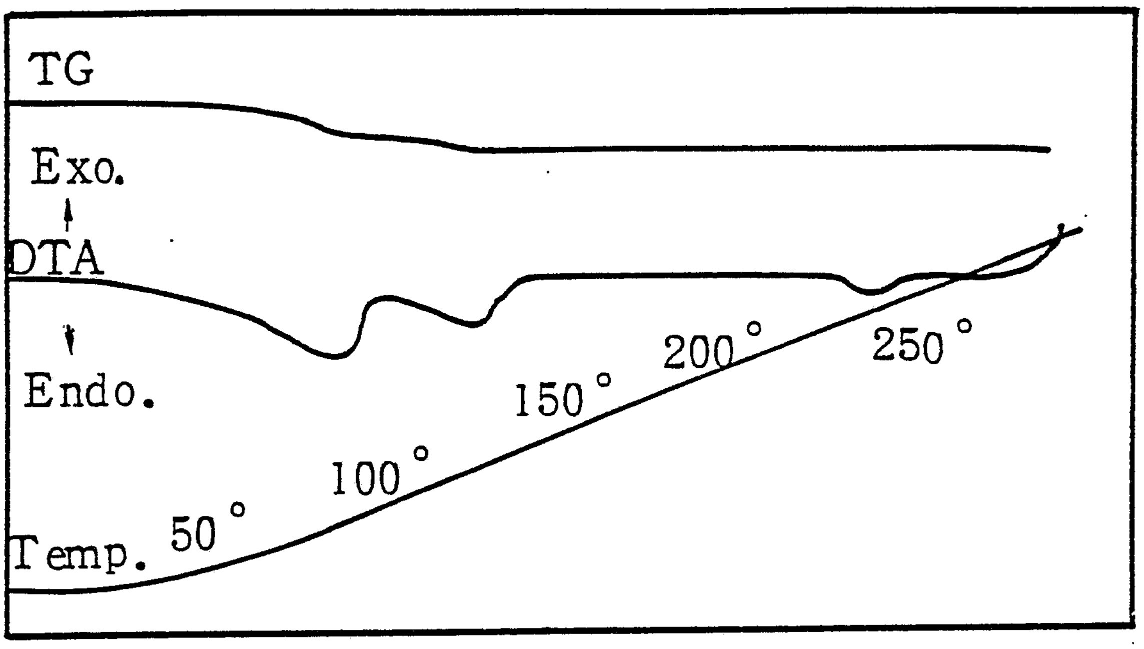

Fig. 4

(B)

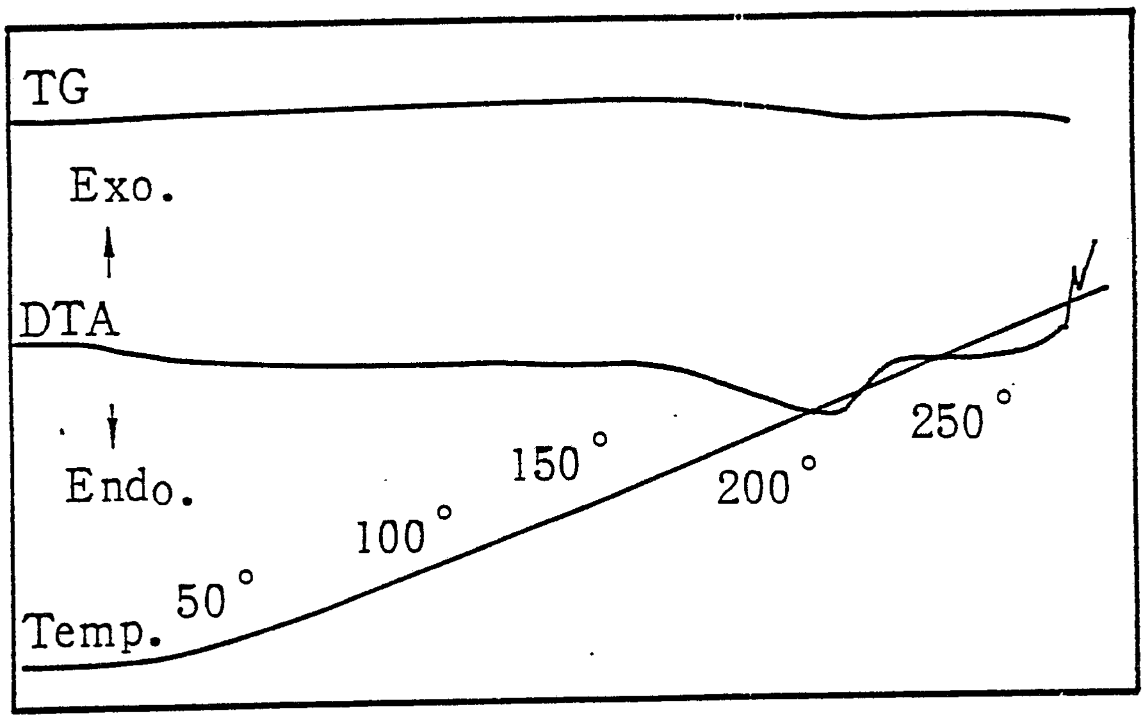

Fig. 4

(C)

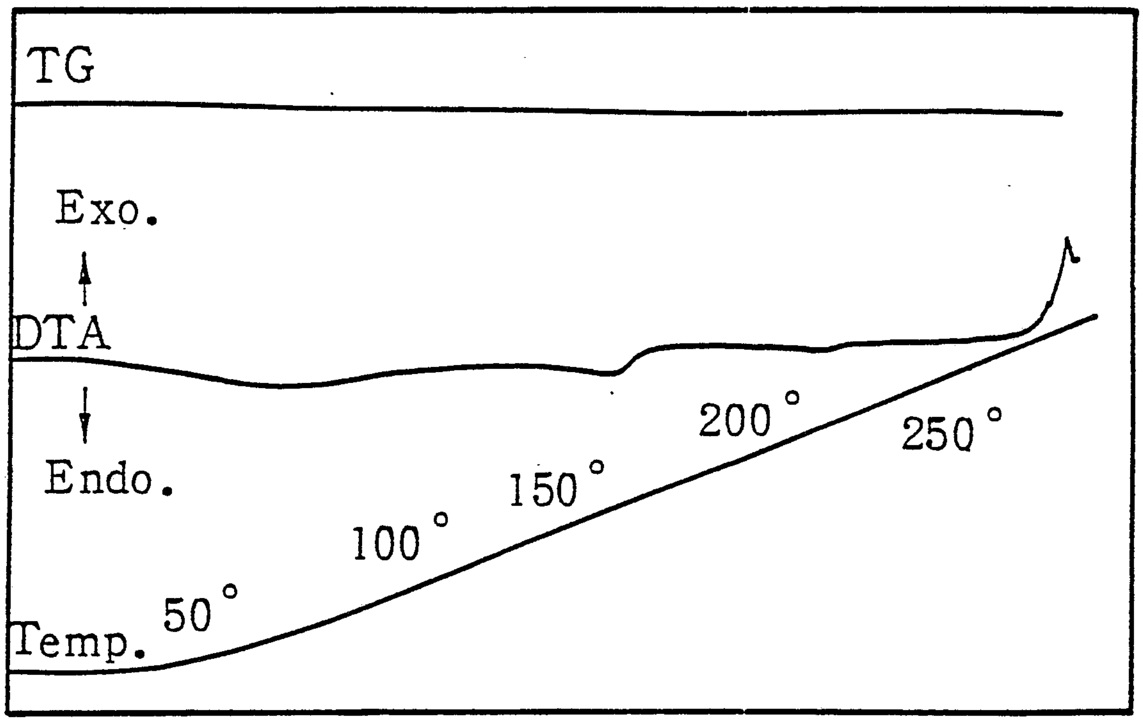

Fig. 5

(A)

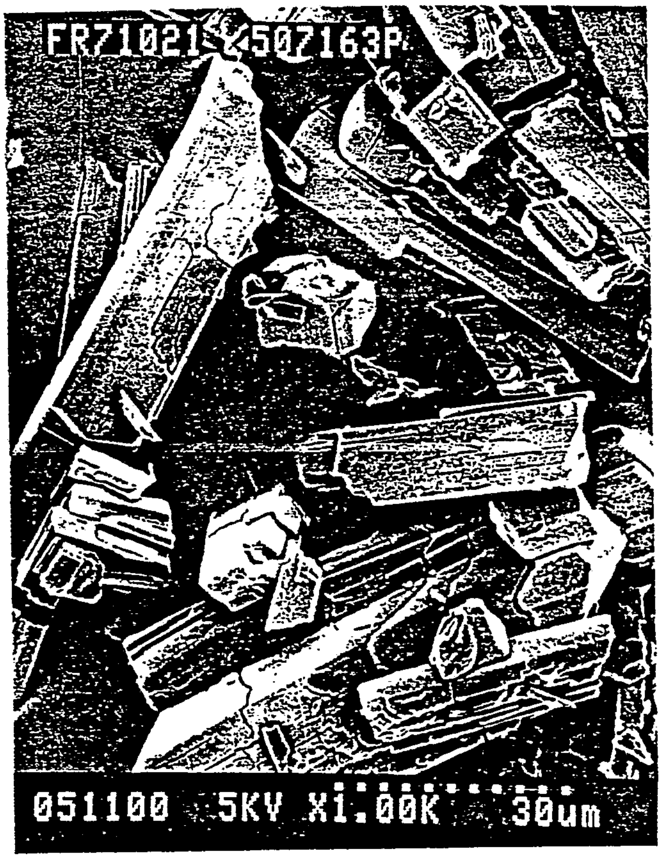

Fig. 5

(B)

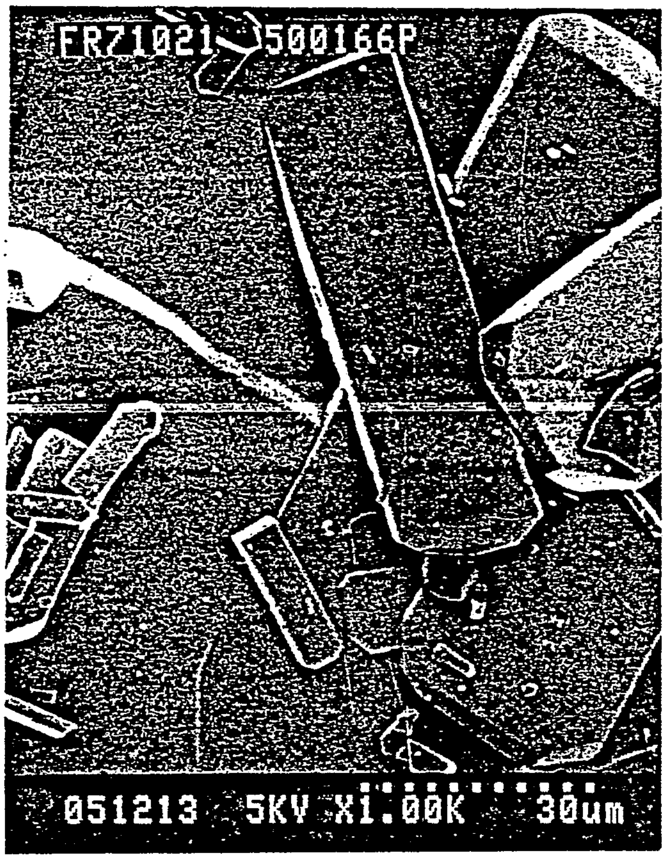

Fig. 5

(C)

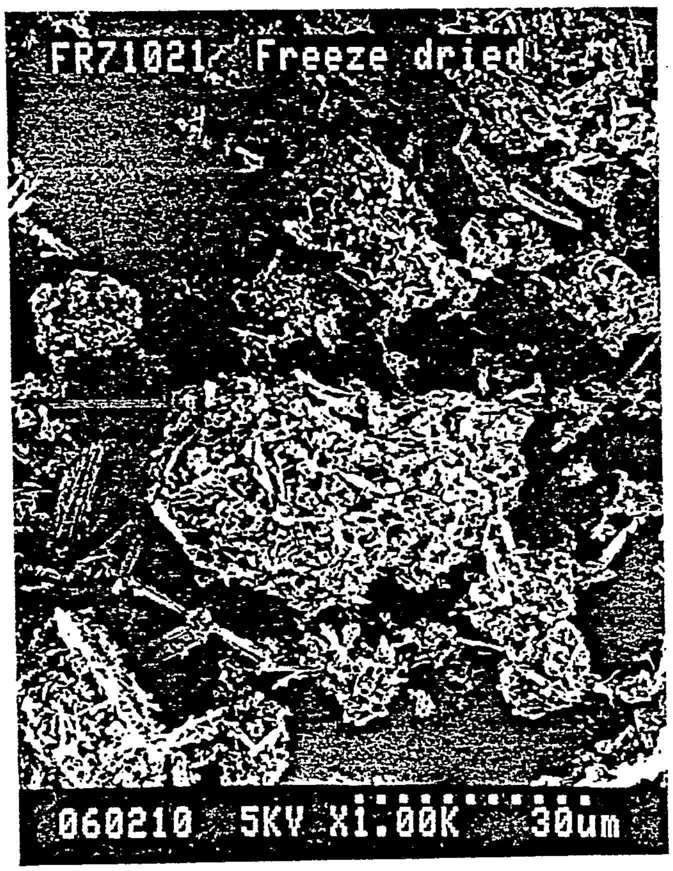

European Patent Office

# EUROPEAN SEARCH REPORT

Application Number

EP 88 11 1995

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,A | EP-A-0 157 346 (FUJISAWA)<br>* Page 75; page 76, line 4; page 82; page 83, line 3 * & US-A-4 650 804<br>----- | 1 | C 07 D 455/02 //<br>A 61 K 31/435 |
| | | | TECHNICAL FIELDS SEARCHED (Int. Cl.4) |
| | | | C 07 D 455/00 |

The present search report has been drawn up for all claims

| Place of search | Date of completion of the search | Examiner |
|---|---|---|
| THE HAGUE | 19-10-1988 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)